Europäisches Patentamt

European Patent Office

Office européen des brevets

(11) Veröffentlichungsnummer: **0 248 117 B1**

# EUROPÄISCHE PATENTSCHRIFT

(45) Veröffentlichungstag der Patentschrift: **19.08.92**

(21) Anmeldenummer: **86116925.8**

(22) Anmeldetag: **05.12.86**

(51) Int. Cl.5: **C23C 4/10,** C23C 4/02, A61F 2/30, A61L 27/00

(54) Verfahren zum Aufbringen einer Schutzschicht auf Gelenkendoprothesen.

(30) Priorität: **03.06.86 DE 3618665**

(43) Veröffentlichungstag der Anmeldung:
**09.12.87 Patentblatt 87/50**

(45) Bekanntmachung des Hinweises auf die Patenterteilung:
**19.08.92 Patentblatt 92/34**

(84) Benannte Vertragsstaaten:
**CH DE FR GB IT LI**

(56) Entgegenhaltungen:
WO-A-86/06617        DE-A- 1 943 801
DE-A- 2 313 678      FR-A- 2 088 764
FR-A- 2 318 617      GB-A- 2 145 117

CHEMICAL ABSTRACTS, Band 95, Nr. 9, November 1981, page 280, Zusammenfassung Nr. 155100t, Columbus, Ohio, US; & GB-A-146 306 (G. BEISTER et al.) 04-02-1981

WT ZEITSCHRIFT FÜR INDUSTRIELLE FERTIGUNG, Band 67, 1977, Seiten 321-325, Springer-Verlag, München, DE; R. SCHARWÄCHTER et al.: "Technik und Anwendung des Plasmaspritzens im Vakuum"

PATENT ABSTRACTS OF JAPAN, Band 4, Nr. 151 (C-28)[633], 23. Oktober 1980, Seite 93 C 28; & JP-A-55 97 464 (MITSUBISHI JUKOGYO K.K.) 24-07-1980

THIN SOLID FILMS, Band 119, Nr. 2, September 1984, Seiten 127-139, Elsevier Sequoia, Lausanne, CH; S.D. BROWN: "The medical-physiological potential of plasma-sprayed ceramic coatings"

(73) Patentinhaber: **AESCULAP AG**
**Möhringer Strasse 125**
**W-7200 Tuttlingen(DE)**

(72) Erfinder: **Winkler-Gniewek, Wladis, Dr. Ing.**
**Brunnentalstrasse 88**
**W-7200 Tuttlingen(DE)**
Erfinder: **Stallforth, Harald, Dr. Ing.**
**Auf dem Schildrain 27**
**W-7200 Tuttlingen(DE)**

(74) Vertreter: **Hoeger, Stellrecht & Partner**
**Uhlandstrasse 14 c**
**W-7000 Stuttgart 1(DE)**

## Beschreibung

Die Erfindung betrifft ein Verfahren zum Aufbringen einer Schutzschicht aus $TiO_2$, $Al_2O_3$, $ZrO_2$ oder $Cr_2O_3$ auf Gleitflächen von Gelenkendoprothesen aus Titan oder Titanlegierungen.

Wegen der notwendigen Körperverträglichkeit werden Endoprothesen häufig aus Titan oder Titanlegierungen hergestellt. Diese Materialien zeigen jedoch ein ungünstiges Verschleißverhalten, das heißt eine schlechte Abriebbeständigkeit. Unter den korrosiven Bedingungen, die im Körper herrschen, ergibt sich außerdem eine Auflösung metallischer Bestandteile der Legierung und damit eine Ansammlung von Korrosionsprodukten im Körper. Auch bei der Paarung derartiger Endoprothesen mit anderen metallischen oder nichtmetallischen Werkstoffen, zum Beispiel Aluminiumoxidkeramik oder Polyethylen, werden metallische Abriebprodukte verstärkt produziert, deren Abfuhr aus der Gelenkkapsel bei künstlichen Gelenken ein Problem darstellt. Es ist sogar bei einem Kontakt mit Knochen mit einem solchen metallischen Abrieb zu rechnen, wenn ausgeprägte Relativbewegungen zwischen einer metallischen Prothesenkomponente und dem Knochen möglich sind, insbesondere im Falle einer Lockerung.

Prothesen aus Titan oder Titanlegierungen lassen sich außerdem mechanisch schlecht bearbeiten, da diese Metalle eine große Neigung zum Verschweißen mit dem Werkzeugmaterial haben und außerdem eine sehr niedrige Wärmeleitfähigkeit aufweisen. Es ist daher nur begrenzt möglich, die für Gleitflächen von künstlichen Gelenken notwendigen Oberflächeneigenschaften zu erreichen.

Es ist bekannt, verschleißbeanspruchte Komponenten aus Titanwerkstoffen mit einer harten Beschichtung zu versehen, beispielsweise auf der Basis von Titannitrid, Titankarbid, Titankarbonitrid oder Titanborid. Solche Beschichtungen werden in der Regel auf chemischem oder physikalischem Wege erzeugt, wobei Schichtdicken im $\mu$m-Bereich bis maximal ca. 50 $\mu$m erreicht werden können. Größere Schichtstärken sind kaum zu erzielen, ohne daß Risse oder Haftungsprobleme auftreten.

Implantate und insbesondere Gelenkkugeln sind auch bereits mit Titanoxid beschichtet worden, und zwar durch eine thermische oder galvanische Behandlung (W. Rostoker, J.O. Galante in: "Biomaterials 1981, Seite 221 bis 224). Durch Oxidation in Gas mit entsprechendem Sauerstoffpotential, zum Beispiel Luft, Sauerstoff, Wasserdampf oder ähnlichem, entsteht bei niedrigen Temperaturen praktisch reines $TiO_2$ an der Oberfläche. Die Schichten sind meistens sehr dünn im Zehntel-Mikrometerbereich. Bei höheren Temperaturen entstehen zwar dickere Schichten, aber auch andere Oxidtypen, eventuell Nitride, deren Wirkungen im Körper nicht bekannt sind. Außerdem neigen sie zum Abplatzen. Eine Überschreitung der Gefügeumwandlungstemperaturen ist wegen der Verschlechterung der mechanischen Eigenschaften des Grundmaterials nicht angebracht. Auf elektrochemischem Wege können zum Beispiel bei der anodischen Oxidation ebenfalls Oxidschichten des Typs $TiO_2$ bis zum 1/10 $\mu$m-Bereich erzielt werden, größere dichte Schichtdicken sind auf diesem Wege nicht erzielbar.

Beschichtungen mit anderen an sich verschleißfesten und biokompatiblen Materialien wie $Al_2O_3$, $ZrO_2$ oder $Cr_2O_3$ können auf diesem Wege nicht erreicht werden.

Es ist auch schon bekannt, auf Endoprothesen eine porige Oberflächenschicht durch Flammspritzen aufzubringen, beispielsweise eine Beschichtung aus Titan, $TiO_2$ oder $Al_2O_3$ (DE-OS 23 13 678). Der Zweck dieser Beschichtung ist jedoch, eine bewußt porige, biokompatible Beschichtung zu erreichen, die das Einwachsen des umgebenden Knochengewebes erleichtert. Eine solche Beschichtung ist allenfalls im Bereich des Prothesenschaftes zu verwenden, sie ist jedoch vollständig ungeeignet zur Beschichtung der Gleitflächen einer Endoprothese, die gerade nicht porig und locker, sondern verdichtet und verschleißfest sein soll.

In der FR-A 2 318 671 ist ein Verfahren beschrieben, um auf einem Implantat aus Titan oder einem anderen Metall eine keramische Oberfläche aufzubringen. Dazu werden in einem Plasmabogen pulverisierte Keramikmaterialien auf die zu beschichtende Oberfläche übertragen. Auf diese Schicht wird bei dem bekannten Verfahren noch Porzellan aufgetragen, das anschließend in einem Tempervorgang bei sehr hohen Temperaturen aufgeschmolzen wird, um das Entstehen von Poren zu vermeiden.

Es ist Aufgabe der Erfindung, ein Verfahren zum Aufbringen einer Schutzschicht auf Gelenkendoprothesen anzugeben, bei dem Beschichtungen mit unterschiedlichen Materialien in einer besonders kompakten Weise auf Gleitflächen von Endoprothesen aufgebracht werden, insbesondere auch in größeren Schichtdicken, wobei die Restporosität gegenüber vergleichbaren Verfahren reduziert wird, so daß Tempervorgänge etc. nicht notwendig werden.

Diese Aufgabe wird bei einem Verfahren der eingangs beschriebenen Art erfindungsgemäß dadurch gelöst, daß man die zu beschichtenden Gleitflächen in einem Vakuum anordnet und auf ihnen die pulverförmigen, in einem heißen, ionisierten Gasstrom (Plasma) aufgeheizten und dadurch anoder aufgeschmolzenem Beschichtungsmaterialien ablagert, daß man Schichtdicken zwischen 0,1 und 1 mm erzeugt, daß man die Beschichtung mechanisch nachbearbeitet und daß man die Oberfläche auf eine mittlere Rauhigkeit unter 0,05 $\mu$m poliert.

Ein solches als Vakuumplasmaspritz-Verfahren bezeichnetes Verfahren ist aus der schweizer Patentschrift 589 145 an sich bekannt. Es wird im Rahmen der vorbekannten Veröffentlichung teilweise für andere Substanzen verwandt, beispielsweise für Stellit.

Das im Rahmen der vorliegenden Erfindung verwendete Vakuumplasmaspritz-Verfahren hat den Vorteil, daß die Beschichtungsmaterialien durch das Aufbringen im Vakuum keine unerwünschten Reaktionen mit Sauerstoff, Stickstoff oder dergleichen eingehen. Außerdem können durch dieses Verfahren außerordentlich kompakte Schichten hergestellt werden, wobei die Kompaktheit durch höhere Pulvergeschwindigkeiten gegenüber vergleichbaren Verfahren in atmosphärischer Umgebung erhöht werden kann. Dadurch wird die Restporosität der Beschichtung gegenüber vergleichbaren Verfahren erheblich reduziert.

Auch die Haftungseigenschaften zwischen der Beschichtung und dem Trägermaterial werden durch dieses Verfahren verbessert, da Interdiffusionsprozesse im Grenzbereich zu einer innigen Verzahnung der Beschichtung mit dem Trägermaterial beitragen. Aufgrund der Vakuumumgebung können die zu beschichtenden Flächen ohne Bedenken bis zur thermischen Stabilitätsgrenze aufgeheizt werden, da keine unerwünschten Reaktionen mit der Umgebung zu befürchten sind. Dadurch wird die Interdiffusion zwischen Substrat und Schicht erhöht. Durch gezielte, zeitliche Temperaturveränderungen können die inneren Spannungen in den aufgespritzten Beschichtungen abgebaut werden.

Es ist mit dem erfindungsgemäßen Verfahren möglich, Schichtdicken zwischen 0,1 und 1 mm zu erzeugen, also Schichtdicken, die wesentlich größer sind als mit herkömmlichen Verfahren erzielte Schichtdicken. Dabei ergeben sich keinerlei Haftungsprobleme, außerdem ist die gesamte Schicht außerordentlich kompakt und porenfrei.

Es ist daher möglich, diese Beschichtung mechanisch nachzuarbeiten, beispielsweise durch Schleifen oder Polieren, so daß man beispielsweise Oberflächen auf eine mittlere Rauhigkeit < 0,05 $\mu$m polieren kann. Dies ist bei den wesentlich kleineren Schichtstärken, die mit bisher verwendeten chemischen, elektrochemischen oder physikalischen Verfahren erzielt worden sind, nicht möglich gewesen.

Zur Erzeugung des ionisierten Gasstromes verwendet man vorzugsweise Argon und Helium.

Besonders vorteilhaft ist es, wenn man die zu beschichtende Fläche vor dem Beschichten durch Sputtern im übertragenen Lichtbogen reinigt. Dadurch werden die Haftungseigenschaften ganz erheblich verbessert.

Es kann weiterhin vorgesehen sein, daß man die Gleitflächen vor der Beschichtung mit $TiO_2$, $Al_2O_3$, $ZrO_2$ oder $Cr_2O_3$ in gleicher Weise mit rein metallischem Titan beschichtet. Eine solche Beschichtung dient als Haftvermittler zwischen dem Trägermaterial einerseits und der Beschichtung mit den Oxiden andererseits. Es ist dabei wesentlich, daß die aufgebrachte Titanzwischenschicht oxidfrei entsteht, da das Verfahren im Vakuum abläuft. Die Zwischenschicht aus rein metallischem Titan kann Stärken bis zu einigen Zehntel Millimetern aufweisen.

Besonders körperverträgliche Beschichtungen ergeben sich, wenn man $TiO_2$ des Typs Rutil oder $Al_2O_3$ des Typs Korund oder $ZrO_2$ des Typs Baddeleyit oder $Cr_2O_3$ des Typs Korund verwendet.

Die nachfolgende Beschreibung einer bevorzugten Ausführungsform der Erfindung dient im Zusammenhang mit der Zeichnung der näheren Erläuterung. Es zeigen:

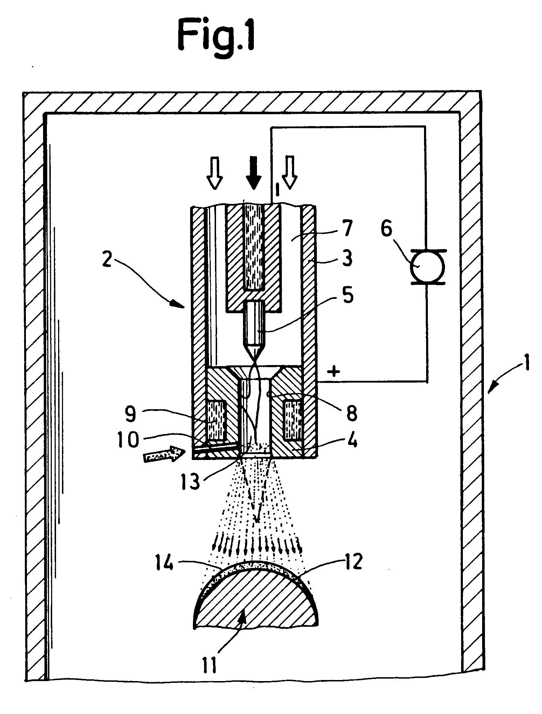

Figur 1: eine schematische Ansicht einer Vorrichtung zur Beschichtung der Gleitfläche einer Gelenkendoprothese und

Figur 2: eine Hüftgelenk-Endoprothese mit einem beschichteten Gelenkkopf.

In einem evakuierbaren Gefäß 1 befindet sich ein Plasmabrenner 2, der im wesentlichen ein äußeres Rohr 3 mit einem Mundstück 4 sowie eine im Inneren des Rohres angeordnete Elektrode 5 aufweist. Die Elektrode 5 besteht aus Wolfram und ist mit einer Spannungsquelle 6 derart verbunden, daß die Elektrode 5 als Kathode geschaltet ist. Sie ist von einem ringförmigen Strömungskanal 7 umgeben, der in einen zentralen Austrittskanal 8 in dem Mundstück 4 mündet. Das Mundstück 4 und das das Mundstück 4 umgebende Rohr 3 sind ebenfalls gemeinsam mit der Spannungsquelle 6 verbunden, diese Teile bilden die Anode der Anordnung.

Der Austrittskanal 8 im Mundstück 4 ist von einer von Kühlwasser durchströmten Kühlleitung 9 umgeben, und in den Austrittskanal 8 tritt in der Nähe von dessen auslaßseitigem Ende eine radiale Zufuhrleitung 10 für ein pulverförmiges Material ein.

In der Verlängerung des Austrittskanales 8 wird eine zu beschichtende Endoprothese 11 derart angeordnet, daß die zu beschichtende Fläche 12 dem Auslaß des Austrittskanals 8 unmittelbar gegenüberliegt.

Im Betrieb wird das Gefäß 1 evakuiert. Der Plasmabrenner wird so betrieben, daß ein inertes Gas, zum Beispiel Argon oder Helium, durch den ringförmigen Strömungskanal 7 und den anschließenden Austrittskanal 8 hindurchströmt. Durch Anlegen einer Spannung zwischen der als Kathode geschalteten Elektrode 5 und dem als Anode geschalteten Mundstück 4, das beispielsweise aus Kupfer besteht, wird ein Lichtbogen 13 zwischen Elektrode 5 und Mundstück 4 erzeugt, so daß das durch den Plasmabrenner 2 strömende Gas den Plasmabrenner als ionisiertes und stark erhitztes Gas (Plasma) verläßt.

Durch die Zufuhrleitung 10 wird in den Gasstrom pulverförmiges Beschichtungsmaterial eingebracht, beispielsweise $TiO_2$. Dieses pulverförmige Beschichtungsmaterial wird von dem Gasstrom mitgerissen, dabei wird das pulverförmige Beschichtungsmaterial oberflächlich an- und eventuell sogar vollständig aufgeschmolzen. Das pulverförmige Beschichtungsmaterial gelangt zusammen mit dem Gasstrom auf die zu beschichtende Fläche 12 und lagert sich dort als Beschichtung 14 ab. Diese Beschichtung ist außerordentlich kompakt und kann beispielsweise eine Schichtdicke bis zu 1 mm erreichen.

Um eine besonders sorgfältige Reinigung der zu beschichtenden Fläche vor Beginn der Beschichtung zu erreichen, ist es möglich, diese Fläche durch Sputtern mittels eines übertragenen Lichtbogens zu reinigen. Dazu wird die Endoprothese selbst als Kathode geschaltet, so daß der Lichtbogen bis zu der zu beschichtenden Fläche ausgebildet wird und somit Verunreinigungen von der zu beschichtenden Fläche entfernt. Nach dieser Reinigung mittels des übertragenen Lichtbogens wird die elektrische Verbindung zu der Gelenkendoprothese wieder unterbrochen. Durch diese Reinigung wird eine optimale Haftung der Beschichtung am Trägermaterial erreicht, insbesondere wird es dadurch möglich, auch auf glatten, vorher nicht aufgerauhten Oberflächen eine fest haftende Beschichtung aufzubringen.

Ein besonderer Vorteil des Beschichtungsverfahrens liegt darin, daß die zu beschichtende Prothese selbst nicht aufgeheizt werden muß, so daß negative Gefügeveränderungen der Prothesenstruktur vermieden werden. Trotzdem ergibt sich durch Interdiffusionsprozesse eine innige Haftung der Beschichtung an der Prothese, selbst bei Dikken der Beschichtung in der Größenordnung bis zu 1 mm.

Nach der Beschichtung kann die Prothese mechanisch nachbearbeitet werden, dabei können mittlere Rauhigkeitswerte < 0,05 μm erreicht werden, es ist auch möglich, Rundheitswerte beispielsweise < 5 μm zu erzielen. Bei Konuskontaktflächen können die Winkelabweichungen < 1' gehalten werden.

Es ist möglich, vor der Beschichtung mit dem Oxid eine rein metallische Titanbeschichtung als Haftvermittlungszwischenschicht aufzubringen. Dazu wird dem Plasmabrenner gegebenenfalls nach Abschluß der Sputterreinigung durch die Zufuhrleitung 10 metallisches Titanpulver zugegeben.

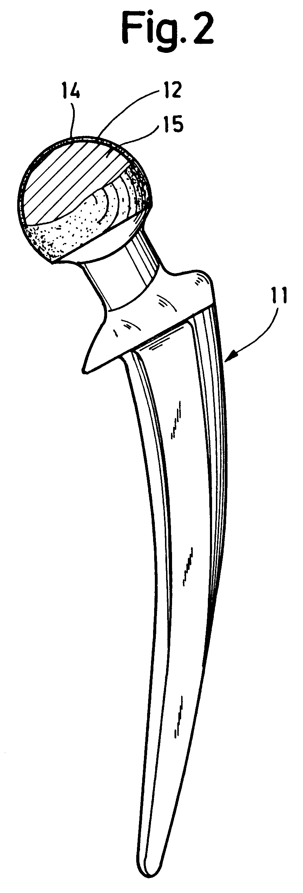

Figur 2 zeigt eine Hüftgelenksendoprothese, deren Gelenkkopf 15 mit einer gemäß dem beschriebenen Verfahren aufgebrachten Beschichtung 14 versehen ist. Der beanspruchte Schutz bezieht sich neben dem Verfahren zum Aufbringen einer solchen Beschichtung auch auf Endoprothesen, die gemäß dem beschriebenen Verfahren mit einer Beschichtung versehen sind.

## Patentansprüche

1. Verfahren zum Aufbringen einer Schutzschicht aus $TiO_2$, $Al_2O_3$, $ZrO_2$ oder $Cr_2O_3$ auf Gleitflächen von Gelenkendoprothesen aus Titan oder Titanlegierungen, **dadurch gekennzeichnet,** daß man die zu beschichtenden Gleitfächen in einem Vakuum anordnet und auf ihnen die pulverförmigen, in einem heißen, ionisierten Gasstrom (Plasma) aufgeheizten und dadurch an- oder aufgeschmolzenen Beschichtungsmaterialien ablagert, daß man Schichtdicken zwischen 0,1 und 1 mm erzeugt, daß man die Beschichtung mechanisch nachbearbeitet und dar man die Oberfläche auf eine mittlere Rauhigkeit unter 0,05 μm poliert.

2. Verfahren nach Anspruch 1, dadurch gekennzeichnet, daß man zur Erzeugung des ionisierten Gasstromes Argon oder Helium verwendet.

3. Verfahren nach einem der voranstehenden Ansprüche, dadurch gekennzeichnet, daß man die zu beschichtende Fläche vor dem Beschichten durch Sputtern im übertragenen Lichtbogen reinigt.

4. Verfahren nach einem der voranstehenden Ansprüche, dadurch gekennzeichnet, daß man die Gleitflächen vor der Beschichtung mit $TiO_2$, $Al_2O_3$, $ZrO_2$ oder $Cr_2O_3$ in gleicher Weise mit metallischem Titan beschichtet.

5. Verfahren nach einem der voranstehenden Ansprüche, dadurch gekennzeichnet, daß man zur Beschichtung $TiO_2$ des Typs Rutil oder $Al_2O_3$ des Typs Korund oder $ZrO_2$ des Typs Baddeleyit oder $Cr_2O_3$ des Typs Korund verwendet.

## Claims

1. A method of applying a protective layer of $TiO_2$, $Al_2O_3$, $ZrO_2$ or $Cr_2O_3$ to sliding surfaces of articulated endoprostheses made of titanium or titanium alloys, characterised in that the sliding surfaces to be coated are arranged in a vacuum and the powdery coating materials are deposited-thereon, which coating materials are heated up in a hot ionised gas flow (plasma) and are thereby melted on, in that layer thicknesses of between 0. 1 and 1 mm are produced, in that the coating is finished mechanically and in that the surface is polished to an average roughness of less than 0.05 $\mu$m.

2. A method according to Claim 1, characterised in that argon or helium is used to produce the ionized gas flow.

3. A method according to any one of the preceding claims, characterised in that the surface to be coated is cleaned before coating by sputtering in the transmitted arc.

4. A method according to any one of the preceding claims, characterised in that, before coating with $TiO_2$, $Al_2O_3$, $ZrO_2$ or $Cr_2O_3$, the sliding surfaces are coated with metallic titanium in a similar manner.

5. A method according to any one of the preceding claims, characterised in that $TiO_2$ of the rutile type or $Al_2O_3$ of the corundum type or $ZrO_2$ of the baddeleyite type or $Cr_2O_3$ of the corundum type are used for coating.

**Revendications**

1. Procédé pour appliquer une couche de protection en $TiO_2$, $Al_2O_3$, $ZrO_2$ ou $Cr_2O_3$ sur les surfaces de glissement des endoprothèses articulaires en titane ou en alliages de titane, caractérisé en ce que l'on dispose les surfaces de glissement à revêtir dans un vide et on dépose sur celles-ci les matériaux de revêtements pulvérulents, chauffés dans un courant gazeux chaud, ionisé (plasma) et de ce fait fondus en surface ou totalement, en ce que l'on forme des épaisseurs de couche comprises entre 0,1 et 1 mm, en ce que l'on soumet le revêtement à un traitement mécanique subséquent et en ce que l'on polit la surface jusqu'à obtenir une rugosité moyenne inférieure à 0,05 $\mu$m.

2. Procédé selon la revendication 1, caractérisé en ce que l'on utilise l'argon ou l'hélium pour former le courant gazeux ionisé.

3. Procédé selon l'une des revendications précédentes, caractérisé en ce que, avant le revêtement, on nettoie par étincelage dans l'arc électrique établi la surface à revêtir.

4. Procédé selon l'une des revendications précédentes, caractérisé en ce que, avant le revêtement avec $TiO_2$, $Al_2O_3$, $ZrO_2$ ou $Cr_2O_3$, on recouvre de la même manière de titane métallique les surfaces de glissement.

5. Procédé selon l'une des revendications précédentes, caractérisé en ce que l'on utilise pour le revêtement $TiO_2$ du type rutile ou $Al_2O_3$ du type corindon ou $ZrO_2$ du type baddeleyite ou $Cr_2O_3$ du type corindon.

# Fig.1

# Fig.2